# EUROPÄISCHE PATENTSCHRIFT

(11) **EP 2 952 873 B1**
(45) Veröffentlichungstag und Bekanntmachung des Hinweises auf die Patenterteilung: **05.08.2020**
(21) Anmeldenummer: 15001648.3
(22) Anmeldetag: 03.06.2015
(51) Int. Cl.: G01N 1/22, B01L 3/00

(54) **GASANALYSEBEUTEL UND VERFAHREN ZUM HERSTELLEN EINES BEUTELS, VERFAHREN ZUM TRANSPORTIEREN / ANALYSIEREN EINER FLUIDPROBE UND VERPACKUNG ZUM VERPACKEN VON FLUIDFÄHIGEM MATERIAL**
GAS ANALYSIS BAG AND METHOD OF MAKING A BAG, METHOD FOR TRANSPORTING/ANALYZING A FLUID SAMPLE AND A PACKAGE FOR PACKAGING FLUID MATERIAL
SACHET D'ANALYSE DE GAZ ET PROCÉDÉ DE FABRICATION D'UN SACHET, PROCÉDÉ DE TRANSPORT / D'ANALYSE D'UN ÉCHANTILLON FLUIDE ET EMBALLAGE DESTINÉ À EMBALLER UN MATÉRIAU POUVANT S'ÉCOULER

(30) Priorität: 03.06.2014 DE 202014004463 U
(43) Veröffentlichungstag der Anmeldung: 09.12.2015
(73) Patentinhaber: Tesseraux Spezialverpackungen GmbH, 68642 Bürstadt/Kreis Bergstraße (DE)
(72) Erfinder: OPITZ, Mirko, 69469 Weinheim (DE)
(74) Vertreter: Grosse Schumacher Knauer von Hirschhausen

(56) Entgegenhaltungen:
- EP-A1- 0 292 439
- WO-A1-03/068134
- WO-A2-2004/026702
- DE-U1- 8 200 215
- DE-U1- 8 900 885
- DE-U1- 20 106 473
- JP-A- 2002 206 996
- US-A- 3 635 092

## Beschreibung

### GEBIET DER ERFINDUNG

Die Erfindung betrifft einen Gasanalysebeutel zum Abfüllen eines fluidfähigen Materials gemäß Anspruch 1. Das fluidfähige Material kann eine Flüssigkeit und/oder ein Gas enthalten oder daraus gebildet sein. Es können auch andere fluidfähige Materiallen vorgesehen sein, etwa ein Granulat. Fluidfähig meint, dass das Material geeignet ist, zu strömen und/oder zu fließen. Der Beutel umfasst eine Beutelwand, die aus zumindest zwei durch zumindest eine Wandverbindung miteinander verbundenen Wandteilen gebildeten Ist. Der Beutel umfasst ferner zumindest einen In der Beutelwand angeordneten, insbesondere flexiblen oder weichen, Beutelschlauch. Durch den Beutelschlauch ist das fluidfähige Material in den Beutel hineinführbar oder aus dem Beutel herausführbar. Die Wandverbindung der Beutelwand umfasst zumindest eine Naht, d. h. einen linien- oder streifenartigen Verbindungsbereich, In dem zum Zweck des Verbindens der Wandteile auf zumindest ein Wandteil eingewirkt wurde.

Die Erfindung betrifft auch eine Verpackung gemäß Anspruch 5. Die Verpackung dient dem Verpacken von fluidfähigem Material respektive Verpackungsinhalt. Insbesondere können mit der Verpackung Flüssigkeiten und/oder Gase verbackt werden oder Flüssigkeitsproben oder Gasproben. Die Verpackung umfasst ein innen hohles Verpackungsgehäuse. Das Verpackungsgehäuse kann Papier und/oder Pappe umfassen oder daraus gebildet sein.

Die Erfindung betrifft ferner Hersteilungsverfahren zum Herstellen hier beschriebenen Beutel nach Ansprüche 6 und 7 sowie ein Verfahren zum Transportieren respektive Analysieren einer Fluidprobe gemäß der Ansprüche 8 respektive 9, wobei eine Fluidprobe eine transport- respektive analysefähige Menge eines zu transportierenden respektive zu analysierenden Fluids Ist.

### TECHNOLOGISCHER HINTERGRUND

Aus zumindest zwei Wandteilen gebildete Beutel sind im Stand der Technik bekannt, etwa aus der DE 201 06 473 U1. Um einen flexiblen Schlauch an dem Beutel anordnen zu können, wird ein Spund oder Stutzen in der Beutelnaht angeordnet. Auf dem Stutzen wird ein Schlauchende etwa durch Schrumpfen, Klemmen oder Stecken angeordnet, so dass der Stutzen einen Adapter zwischen dem flexiblen Schlauch und der Beutelwand bildet. Es betreffen somit mehrere Herstellungsschritte das Anordnen des Spundes und das Anordnen des flexiblen Schlauchs an dem Beutelspund. Hier setzt die Erfindung ein.

### DARSTELLUNG DER ERFINDUNG

Die Aufgabe besteht darin, die Herstellung und Handhabung entsprechender Beutel im Hinblick auf Kosten und apparativen Aufwand, sowie im Hinblick auf das Abfüllen und/oder das Verpacken entsprechender Materialien oder Stoffe, so zu vereinfachen, dass eine (Fluid-)Dichtigkeit des Beutels nicht beeinträchtigt wird. Diese Aufgabe wird durch einen Beutel nach Anspruch 1 gelöst. Demnach ist der Beutelschlauch zwischen zwei Wandteilen in der Naht fluiddicht angeordnet. Das Anordnen des Schlauchs an dem Beutel erfolgt demnach unmittelbar, ein Adapter oder ein Spund Ist nicht erforderlich. Sämtliche Herstellungsschritte, die ein Anordnen des Schlauchs auf dem Spund betreffen, entfallen bei dem erfindungsgemäßen Beutel, wodurch gegenüber dem Stand der Technik eine erhebliche Vereinfachung und Kostenersparnis erzielt wird. Der weiche und/oder flexible Schlauch wird direkt an dem Beutel angeordnet, nämlich zwischen dem ersten und dem zweiten Wandteil der Beutelwand. Die Wandteile können dabei durch thermischen Energieeintrag miteinander verbunden sein, etwa durch Heißsiegeln oder durch Schweißen. Es kann auch vorgesehen sein, dass die Wandteile durch Kleben miteinander verbunden sind. Der Beutel kann als Folienbeutel gebildet sein, d. h. die Wandteile werden aus einer Folle oder aus Folien gebildet. Die Wandteile können von einer Folienbahn abgetrennt sein oder aus einer Folie ausgestanzt sein. Sofern der Beutel als Folienbeutel gebildet ist, Ist er günstig und mit geringem apparativen Aufwand herstellbar, einfach (weiter-)verarbeitbar, einfach transportierbar (leicht), einfach zu falten (und zu verpacken) und somit insgesamt einfach handhabbar.

Die Beutelwand ist aus einer, insbesondere mehrlagigen, Folie gebildet. Die Folie, aus der die Beutelwand oder zumindest ein Wandteil der Beutelwand gebildet Ist, kann Polyester, insbesondere Polyethylenterephthalat (PET), umfassen und/oder einen thermoplastischen Kunststoff, insbesondere Polyethylen (PE). Die Folienbestandteile sind in an sich bekannter Welse verarbeitbar und günstig zu beschaffen. Das Material, aus dem der Schlauch gebildet ist, kann derart weich sein, dass der Schlauch mit einer, insbesondere auf die äußere Mantelfläche des Schlauchs wirkenden, Presskraft zusammendrückbar ist. Duch das zusammendrücken kann der Schlauch verschließbar sein. Die Presskraft dabei ist geringer als 100 N, insbesondere geringer als 10 N. Beispielsweise kann die Presskraft durch zwei Finger einer Hand aufgebracht werden. Alternativ kann der Schlauch durch Aufbringen einer gergleichsweis gegeringen Saugkraft zusammenquetschbar sein. Der weiche Schlauch wird zwischen den Wandteilen der Beutelwand durch Heißsiegeln angeordnet, wodurch der Beutel im Bereich der den Schlauch fixierenden Siegelnaht durch den weichen Schlauch keine oder eine kaum spürbare Verstärkung aufweist. Es kann vorgesehen sein, dass die Beutelwand eine äußere Schicht aus Polyester, insbesondere Polyethylenterephthalat (PET), umfasst, was die Folie beständiger und stabiler werden lässt,

Die Beutelwand kann eine zur Beutelinnenseite zugewandte, innere Schicht aus einem thermoplastischen Kunststoff, insbesondere Polyester (PE), umfassen, wobei die Wandverbindung eine Kunststoff-Siegelnaht umfasst. Der Beutelschlauch umfasst ein Insbesondere thermoplastisches Elastomer (TPE) oder Polypropylen (PP) oder Polyolefin oder ein Styrol-Ethylen-Butylen-Blockcopolymeren (SEB) oder Kombinationen daraus. Auf diese Weise wird erreicht, dass die Wandteile der Beutelwand auf einfache und dennoch (fluid-)dichte Weise miteinander durch Heißsiegeln verbindbar sind. Hierbei wird eine PE-auf-PE-Siegelnaht gebildet. Der Beutelschlauch kann In oder an der Naht durch Heißsiegeln angeordnet sein. Insbesondere dort, wo der Schlauch In der Naht angeordnet ist, wird die PE-Schicht der Folie respektive werden die PE-Schichten der Folien mit dem Schlauch durch Heißsiegeln so miteinander verbunden, dass eine fluiddichte PE-auf-TPE-Siegelnaht, eine fluiddichte PE-auf-PP-Siegelnaht, oder eine fluiddichte PE-auf-SEB-Siegelnaht gebildet ist.

Zur Verbesserung der thermischen Stabilität und zur Verbesserung der Dichtigkeit der Beutelwand kann eine Barriereschicht vorgesehen sein, Insbesondere aus elnem Metall, bevorzugt aus Aluminium. Es kann dabei auch vorgesehen sein, dass eine oder mehrere Folienschichten mit einem oder mehreren Haftvermittlern ausgestattet sind.

Zum Verschließen respektive Wiederverschließen des Beutels kann ein Verschluss vorgesehen sein, der bevorzugt In oder an dem Beutelschlauch anordbar ist. Der Verschluss kann einen Stöpsel oder eine Klemme umfassen. Es kann auch ein Verschluss vorgesehen sein, bei dem ein Knicken des Schlauchs vorgesehen ist, und/oder bei dem durch Kleben der Schlauch abgedichtet wird.

Der Beutel ist als Doppelbeutel mit zwei Beutelsegmenten oder als Mehrfachbeutel mit mehreren Beutelsegmenten gebildet, wobei jedem Beutelsegment jeweils ein Beutelschlauch zugeordnet ist. So lassen sich unterschiedliche Fluide oder mehrere Proben eines Fluids in einem Doppel- oder Mehrfachbeutel kombiniert transportieren.

Eine Beeinträchtigung der Dichtigkeit des hier beschriebenen Beutels ist auch dann nicht gegeben, wenn der Beutel als einfach zu handhabender Gasanalysebeutel verwendet wird, wobei das fluidfähige Material aus einem Gas gebildet ist. Das Gas wird über den Beutelschlauch in den Beutel hineingefördert. Beutel und Beutelwand sind gasdicht oder nahezu gasdicht. Nahezu gasdicht meint, dass der Beutel so beschaffen ist, dass die Gasprobe über einen angemessenen Zeitraum, d. h. über mehrere Tage oder Wochen, in dem Beutel nahezu vollständig verbleibt und etwaige Verluste in Verbindung mit einem Leck in diesem Zeitraum vernachlässigbar sind. Im Übrigen ist der Beutel, insbesondere im Bereich des Beuteischlauchs, verschließbar.

Maßnahmen zum Vereinfachen des Abfüllens und/oder der Verpackens entsprechender Materialien oder Stoffeergeben sich ferner durch eine Verpackung nach Anspruch 9. Demnach dient die Verpackung zum Verpacken von fluldfählgem Material, insbesondere zum Verpacken von Flüssigkeiten und/oder Gasen und/oder Flüssigkeits- und/oder Gasproben. Die Verpackung umfasst ein insbesondere Papier und/oder Pappe umfassendes Verpackungsgehäuse. In dem inneren Hohlraum des Verpackungsgehäuses ist zumindest ein Beutel unverlierbar angeordnet. Das Verpackungsgehäuse kann eckig gebildet sein und es umgibt den Beutel. Beispielsweise kann das Verpackungsgehäuse quaderförmig sein oder Würfelförmig oder zylindrisch oder tetraedisch. Der in dem verpackungsgehäuse angeordnete Beutel umfasst eine aus zumindest zwei durch zumindest eine Wandverbindung miteinander verbundenen Wandteilen gebildeten Beutelwand. Bei dem Beutel kann es sich um einen Gasanlysebeutel handeln. Der Beutel umfasst zumindest einen in der Beutelwand angeordneten flexiblen Beutelschlauch, durch den das fluidfähige Material in den Beutel hineinführbar oder aus dem Beutel herausführbar ist. Die Wandverbindung umfasst zumindest eine Naht. Der flexible Beutelschlauch ist zwischen zwei Wandteilen in der Naht fluiddicht angeordnet. Die Beutelwand ist aus einer, insbesondere mehrlagigen, Folie gebildet. Die Wand ist aus einer Polyester, nämlich Polyethylenterephthalat (PET), und einer einen thermoplastischen Kunststoff, nämlich Polyethylen (PE), umfassenden Folie gebildet. Der Beutelschlauch ist aus einem thermoplastischen Elastomer (TPE) gebildet und umfasst vorzugsweise Polypropylen (PP) oder Polyolefin oder ein Styrol-Ethylen-Butylen-Blockcopolymer (SEB) oder Kombinationen daraus.

Die hier beschriebene Verpackung wird gelegentliche auch als Bag-in-Box-Verpackung bezeichnet. Die in der Verpackung zu transportierende Flüssigkeit oder das zu transportierende Gas oder die zu transportierende Gasprobe wird durch das den Beutel umgebende Verpackungsgehäuse zusätzlich geschützt. Außerdem kann das Verpackungsgehäuse besser beschriftet oder Ettikettiert werden als etwa der befüllte und dadurch gewölbte Beutel.

Eine Vereinfachung bei dem Herstellen respektive beim Handhaben der hier beschriebenen Beutel ergibt sich ferner durch ein Verfahren zum Herstellen eines Beutels nach Anspruch 11, Bei dem Herstellungsverfahren ist vorgesehen, dass ein erstes, eine PE-Schicht umfassendes Wandteil mit zumindest einem zweiten, eine PE-Schicht umfassenden Wandteil zu einer Beutelwand durch Heißsiegeln der PE-Schichten der Wandteile entlang einer eine Wandverbindung bildenden Siegelnaht verbunden wird. Durch eine im Bereich der Wandverbindung angeordnete Schlauchverbindung wird ein, insbesondere flexibler, Insbesondere aus einem verglichen mit dem Material der Wandteile weichen Schlauchmaterial gebildeter, Beutelschlauch mit der PE-Schicht der ersten und/oder mit der PE-Schicht der zweiten Beutelwand durch Heißsiegeln verbunden. Der Beutel ist, insbesondere im Bereich Wandverbindung und im Bereich der Schlauchverbindung, gasdicht oder nahezu gasdicht. Ein Anordnen des Schlauchs an einem Adapter oder Ähnlichem ist nicht erforderlich, wodurch gegenüber dem Stand der Technik mehrere aufwändige Herstellungsschritte entfallen.

Die Wandverbindung kann Im Bereich der Schlauchverbindung so unterbrochen sein, dass die Wandverbindung im Bereich der Schlauchverbindung durch die Schlauchverbindung ersetz wird. Der Schlauch kann ein insbesondere thermoplastisches Elastomer (TPE) oder Polypropylen (PP) oder Polyolefin oder ein Styrol-Ethylen-Butylen-Blockcopolymeren (SEB) oder Kombinationen daraus umfassen.

Eine vereinfachte Handhabung des hier beschriebenen Beutels ohne Beeinträchtigung seiner Dichtigkeit ergibt sich ferner durch ein Verfahren zum Transportieren einer Fluidprobe, insbesondere einer Gasprobe nach Anspruch 14. Die Fluidprobe wird in einen hier beschriebenen Beutel oder in eine hier beschriebene Verpackung eingefüllt. Anschließend wird der Beutel verschlossen, so dass die Fluidprobe in dem verschlossenen und (nahezu) gasdichten Beutel transportierbar ist.

Eine vereinfachte Handhabung des hier beschriebenen Beutels ohne Beeinträchtigung seiner Dichtigkeit ergibt sich schließlich durch ein Verfahren zum Analysieren einer Fluidprobe nach Anspruch 15. Dabei wird zunächst eine Fluidprobe erzeugt oder beschafft. Die Fluidprobe wird In einen hier beschriebenen Beutel (hier beschriebene Verpackung) eingefüllt. Der befüllte Beutel wird zu einer Analysevorrichtung transportiert, Insbesondere gemäß einem hier beschriebenen Transportverfahren. Ein In dem Beutel angeordneter Beutelschlauch wird mit der Analysevorrichtung fluidtechnisch verbunden, so dass der Fluidinhalt des Beutels der Analysevorrichtung zuführbar ist. Das Fluid ist in der Fluidvorrichtung in an sich bekannter Weise analysierbar.

Die vorgenannten sowie die beanspruchten und In den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können

Weitere Einzelheiten, Merkmale und Vorteile des Gegenstandes der Erfindung ergeben sich aus den Unteransprüchen, sowie aus der nachfolgenden Beschreibung und der zugehörigen Zeichnung, In der - beispielhaft - ein Ausführungsbeispiel eines Beutels dargestellt Ist Auch einzelne Merkmale der Ansprüche oder der Ausführungsformen können mit anderen Merkmalen anderer Ansprüche und Ausführungsformen kombiniert werden.

### KURZBESCHREIBUNG DER FIGUREN

In der Zeichnung zeigen
- Fig. 1: eine Aufsicht auf einen Horizontalschnitt durch einen Beutel,
- Fig. 2: eine Aufsicht auf einen Horizontalschnitt durch einen Doppelbautel.

### DETAILLIERTE BESCHREIBUNG VON AUSFÜHRUNGSBEISPIELEN

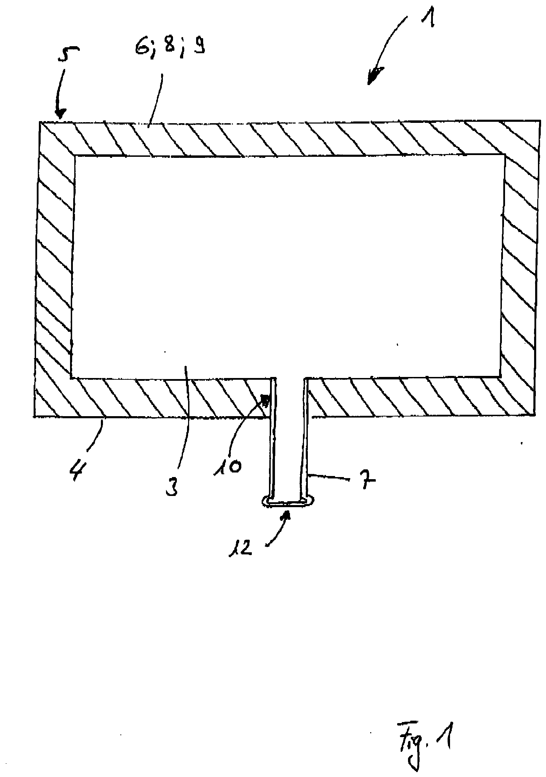

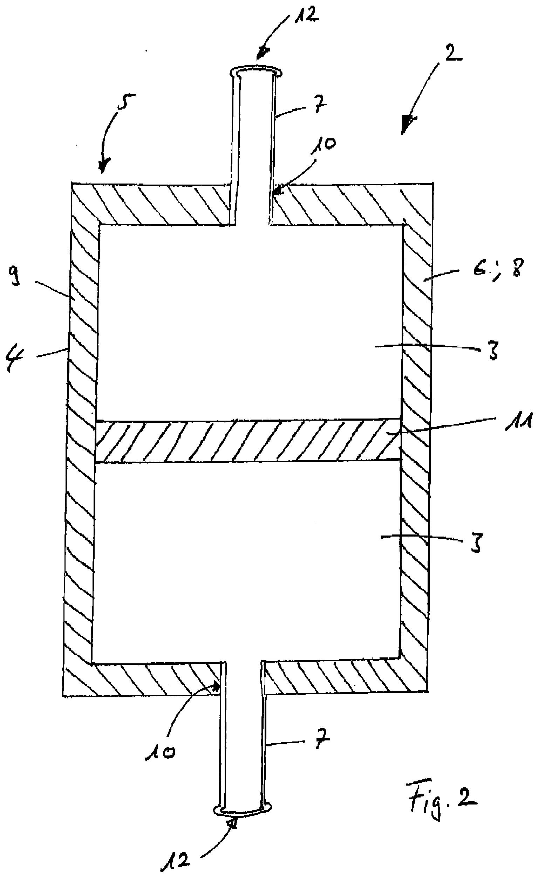

Die Figuren 1 und 2 zeigen Gasanalysebeutel 1, 2, wobei der Fig. 2 ein Doppelbeutel 2 mit zwei Beutelsegmenten 3 entnommen werden kann. Die Fig. 1 zeigt einen (Einfach-)Beutel 1 mit nur einem Beutelsegment 3. Die Gasanalysebeutel 1, 2 gemäß der Figuren 1 und 2 sind dazu geeignet, dass in ihnen ein fluidfähiges Material, nämlich ein Gas, abfüllbar ist. Bei dem Gas kann es sich beispielsweise um (Atem-)Luft handeln, die einer (Atem-)Luftanalyse zugeführt werden soll, und die zu Transportzwecken in dem Gasanalysebeutel 1, 2 abgefüllt wird. Die Gas- respektive Luftprobe kann so einer Analyse zugeführt werden, wobei das Befüllen des Gasanalysebeutels 1, 2 und das Analysieren der Gasprobe an unterschiedlichen Orten erfolgen kann.

Alternativ können In die Beutel 1, 2, wie sie den Figuren 1 und 2 zu entnehmen sind, auch Flüssigkeitsproben eingefüllt werden, die einer Analyse zugeführt werden können.

Der Beutel 1 gemäß Fig. 1 umfasst eine Beutelwand 4, die aus zwei Wandteilen 5 gebildet ist, Der Beutel gemäß Fig. 1 umfasst ein oberes und ein unteres Wandteil 5, wobei in der Flg. 1 nur das untere Wandteil 5 In einer entlang einer Wandverbindung 6 geschnitten dargesteilten Aufsicht abgebildet ist. Die Wandteile 5 werden entlang der Wandverbindung 6 miteinander verbunden. Das Verbinden der Wandteile 5 miteinander erfolgt durch eine Heißsiegelnaht 8, die im Bereich des Rands 9 der Wandteile 5 angeordnet ist. Die Heißsiegelnaht 8 ist gasdicht oder nahezu gasdicht, so dass der Beutelinhalt nicht oder nur sehr langsam aus dem Beutelvolumen entweichen kann.

Der Beutel gemäß Fig. 1 umfasst einen Schlauch 7, der in einer (randseitigen) Naht 8 der Wandverbindung 6 angeordnet ist. Der Schlauch 7 ist durch Heißsiegeln mit dem (oberen und dem unteren) Wandteil 5 verbunden. Das Verbinden des Schlauchs 7 mit den Wandteilen 5 erfolgt Im Bereich der Schlauchverbindung 10. An der Stelle, wo die Schlauchverbindung 10 angeordnet ist, sind (das obere und das untere) Wandteil 5 nicht unmittelbar miteinander verbunden, die Wandverbindung 6 wird an dieser Stelle durch die Schlauchverbindung ersetz. Dadurch wird erreicht, dass der Schlauch 7 gasdicht in dem Beutel 1 angeordnet ist. Es kann demnach auch im Bereich der Schlauchverbindung 10 kein Gas oder nahezu kein Gas aus dem Beutelvolumen entweichen.

Die Wandteile 5 des Beutels 1 sind aus einer mehrlagigen Kunststofffolie gebildet, wobei außenseitig eine stabile PET-Lage vorgesehen ist und Innenseitig eine PE-Lage. Die PE-Lage ist demnach auf der Seite der Wandteile 5 der Beutelwand, die mit dem Beuteilnhalt In Kontakt tritt. Zwischen der PET-Lage und der PE-Lage ist eine Aluminium-Lage angeordnet, wobei zwischen der Aluminium-Lage und zumindest einer der benachbarten Folienlagen ein Haftvermittler vorgesehen sein kann.

Die Wandverbindung 6 umfasst eine Heißsiegelnaht 8. Die aufeinander angeordneten PE-Lagen des Folienverbunds der Wandteile 5 der Wand 4 des Beutels 1 werden, etwa durch Heißsiegeln respektive durch thermischen Energieeintrag, stoffschlüssig miteinander verbunden, wodurch die Wandverbindung gasdicht oder nahezu gasdicht ist.

Der Schlauch 7 ist aus einem thermoplastischen Elastomer gebildet. Im Bereich der Schlauchverbindung 10 wird, etwa durch Heißsiegeln respektive durch thermischen Energieeintrag, eine gasdichte oder nahezu gasdichte Verbindung mit der PE-Lage des Folienverbunds der Wandteile 5 erzielt, wodurch auch die Schlauchverbindung 10 gasdicht oder nahezu gasdicht ist. Im Bereich der Schlauchverbindung 10 ist die PE-Lage eines Wandteiles 5 daher nicht mit der PE-Lage des anderen Wandteils 5 verbunden sondern mit dem Elastomer des Schlauchs 7. Ein Adapter zwischen Schlauch 7 und Beutelwand 4 ist nicht erforderlich, ebenso wenig ein aufwändiges Aufstecken des Schlauchs auf einen Adapter.

Die Fig. 2 zeigt einen Doppelbeutel 2, d. h. einen Beutel 2 mit zwei räumlich voneinander durch eine Siegeltrennung 11 getrennten Beutelsegmenten 3. Jedem Beutelsegment 3 Ist ein Schlauch 7 zugeordnet, so dass jedes Beutelsegment 3 durch einen eigenen Schlauch 7 befüllt respektive entleert werden kann. In dem Beutel 2 gemäß Fig. 2 können in den Beutelsegmenten 3 beispielsweise unterschiedliche Gasproben eingebracht werden, die dann unabhängig voneinander untersucht werden können. Es kann auch vorgesehen sein, dass in dem einen Beutelsegment 3 eine Referenzprobe eingebracht wird, die mit der Gasprobe des anderen Beutelsegments 3 bei der Analyse vergleichbar ist.

Für eine Analyse wird ein Beutelsegment 3 eines Beutels 1, 2 mit einem Gas über den Schlauch 7 befüllt. Anschließend wird der Beutel 1, 2 verschlossen. Das Verschließen des Beutels 1, 2 kann über einen Stopfen erfolgen, der in der Öffnung 12 des Schlauchs angeordnet wird. Alternativ kann zum Verschließen des Beutels 1, 2 ein Abklemmen des Schlauchs 7 mittels einer Klemmvorrichtung erfolgen. Der mit der Gasprobe befüllte und verschlossene Beutel 1, 2 ist dann transportfähig und kann, etwa per Post oder per Kurier zu einer Analysevorrichtung transportiert werden. Dort wird der Beutel 1, 2, etwa mit dem Schlauch 7, an die Analysevorrichtung angeschlossen, so dass die Gasprobe respektive die Gasproben aus dem Beutel entnommen und analysiert werden können.

### BEZUGSZEICHENLISTE

- 1: Beutel
- 2: Doppelbeutel
- 3: Beutelsegment
- 4: Beutelwand
- 5: Wandteil
- 6: Wandverbindung
- 7: Schlauch
- 8: Naht
- 9: Rand
- 10: Schlauchverbindung
- 11: Siegeltrennung
- 12: Öffnung

## Patentansprüche

1. Gasanalysebeutel (1, 2) zum Abfüllen eines fluidfähigen Materials, wobei der Beutel (1, 2) eine aus zumindest zwei durch zumindest eine Wandverbindung (6) miteinander verbundenen Wandteilen (5) gebildeten Beutelwand (4) umfasst, sowie zumindest einen in der Beutelwand (4) angeordneten flexiblen Beutelschlauch (7), durch den das fluidfähige Material in den Beutel (1, 2) hineinführbar oder aus dem Beutel (1, 2) herausführbar ist, wobei die Wandverbindung (6) zumindest eine Kunststoff-Siegelnaht (8) umfasst, wobei die Beutelwand (4) aus einer mehrlagigen Folie gebildet ist, wobei die Beutelwand (4) eine äußere Schicht aus Polyethylenterephthalat (PET) umfasst und eine innere Schicht aus einem thermoplastischen Kunststoff, nämlich Polyethylen (PE), wobei der flexible Beutelschlauch (7) zwischen zwei Wandteilen (5) in der Naht (8) fluiddicht angeordnet ist, wobei der Beutelschlauch (7) aus einem verglichen mit dem Material der Wandteile weichen Schlauchmaterial gebildet ist, nämlich aus einem thermoplastischen Elastomer (TPE), wobei der Beutelschlauch (7) in der Naht (8) durch Heißsiegeln angeordnet ist, nämlich durch eine PE-auf-TPE-Siegelnaht, und wobei das Material, nämlich ein thermoplastisches Elastomer (TPE), aus dem der Schlauch gebildet ist, derart weich ist, dass der Schlauch mit einer auf die äußere Mantelfläche des Schlauchs wirkenden Presskraft den Schlauch verschließend zusammendrückbar ist, wobei die Presskraft geringer als 10 N ist, sodass die Presskraft durch zwei Finger einer Hand aufbringbar ist,
und wobei der Beutel als Doppelbeutel (2) mit zwei Beutelsegmenten (3) oder als Mehrfachbeutel mit mehreren Beutelsegmenten (3) gebildet ist, wobei jedem Beutelsegment (3) jeweils ein Beutelschlauch (7) zugeordnet ist.

2. Gasanalysebeutel (1, 2) nach Anspruch 1 wobei der Beutelschlauch (7) ein Styrol-Ethylen-Butylen-Blockcopolymeren (SEB) umfasst.

3. Gasanalysebeutel (1, 2) nach Anspruch 1 oder 2, wobei die Beutelwand (4) eine Barriereschicht, insbesondere aus einem Metall, bevorzugt aus Aluminium, umfasst.

4. Gasanalysebeutel (1, 2) nach einem der Ansprüche 1 bis 3, mit einem, insbesondere eine Presskraft aufbringenden, Verschluss, der bevorzugt in oder an dem Beutelschlauch (7) angeordnet ist.

5. Verpackung zum Verpacken von fluidfähigem Material, mit einem Verpackungsgehäuse, welches insbesondere Papier und/oder Pappe umfasst, und mit zumindest einem in dem Verpackungsgehäuse unverlierbar angeordneten Beutel (1, 2), nach einem der Ansprüche 1 bis 4.

6. Verfahren zum Herstellen eines Beutels nach Anspruch 1 oder nach Anspruch 3, wenn abhängig von Anspruch 1, oder nach Anspruch 4, wenn abhängig von einem der Ansprüche 1 oder 3, bei dem ein erstes, eine PE-Schicht umfassendes Wandteil mit zumindest einem zweiten, eine PE-Schicht umfassenden Wandteil zu einer Beutelwand durch Heißsiegeln der PE-Schichten der Wandteile entlang einer eine Wandverbindung bildenden Siegelnaht verbunden wird, wobei die Beutelwand (4) eine äußere Schicht aus Polyethylenterephthalat (PET) umfasst und eine innere Schicht aus PE, bei dem durch eine im Bereich der Wandverbindung angeordnete Schlauchverbindung ein flexibler aus einem verglichen mit dem Material der Wandteile weichen Schlauchmaterial gebildeter, Beutelschlauch mit der PE-Schicht der ersten und/oder mit der PE-Schicht der zweiten Beutelwand durch Heißsiegeln verbunden wird, so dass der Beutel, insbesondere im Bereich Wandverbindung und im Bereich der Schlauchverbindung, gasdicht oder nahezu gasdicht ist, wobei der Schlauch aus einem thermoplastischen Elastomer (TPE) gebildet ist, und wobei die Wandverbindung im Bereich der Schlauchverbindung so unterbrochen ist, dass die Wandverbindung im Bereich der Schlauchverbindung durch die Schlauchverbindung ersetz wird.

7. Verfahren zum Herstellen eines Beutels nach Anspruch 2 oder nach Anspruch 3, wenn abhängig von Anspruch 2, oder nach Anspruch 4, wenn abhängig von einem der Ansprüche 2 oder 3, bei dem ein erstes, eine PE-Schicht umfassendes Wandteil mit zumindest einem zweiten, eine PE-Schicht umfassenden Wandteil zu einer Beutelwand durch Heißsiegeln der PE-Schichten der Wandteile entlang einer eine Wandverbindung bildenden Siegelnaht verbunden wird, wobei die Beutelwand (4) eine äußere Schicht aus Polyethylenterephthalat (PET) umfasst und eine innere Schicht aus PE, bei dem durch eine im Bereich der Wandverbindung angeordnete Schlauchverbindung ein flexibler aus einem verglichen mit dem Material der Wandteile weichen Schlauchmaterial gebildeter, Beutelschlauch mit der PE-Schicht der ersten und/oder mit der PE-Schicht der zweiten Beutelwand durch Heißsiegeln verbunden wird, so dass der Beutel, insbesondere im Bereich Wandverbindung und im Bereich der Schlauchverbindung, gasdicht oder nahezu gasdicht ist, wobei der Schlauch aus einem thermoplastischen Elastomer (TPE) gebildet ist und ein Styrol-Ethylen-Butylen-Block-copolymeren (SEB) umfasst, und wobei die Wandverbindung im Bereich der Schlauchverbindung so unterbrochen ist, dass die Wandverbindung im Bereich der Schlauchverbindung durch die Schlauchverbindung ersetz wird.

8. Verfahren zum Transportieren einer Fluidprobe, insbesondere einer Gasprobe, bei dem die Fluidprobe in einen Beutel nach einem der Ansprüche 1 bis 4 oder in eine Verpackung nach Anspruch 5 eingefüllt wird, und bei dem der Beutel anschließend verschlossen wird, so dass die Fluidprobe in dem verschlossenen Beutel transportierbar ist.

9. Verfahren zum Analysieren einer Fluidprobe, bei dem eine Fluidprobe erzeugt oder beschafft wird, bei dem die Fluidprobe in einen Beutel nach einem der Ansprüche 1 bis 4 oder in eine Verpackung nach Anspruch 5 eingefüllt wird, bei dem der befüllte Beutel zu einer Analysevorrichtung, gemäß einem Verfahren nach Anspruch 8, transportiert wird, bei dem ein in dem Beutel angeordneter Beutelschlauch mit der Analysevorrichtung fluidtechnisch verbunden wird, so dass das Fluid der Analysevorrichtung zuführbar ist, und bei dem das Fluid in der Fluidvorrichtung analysierbar ist.

## Claims

1. A gas analysis bag (1, 2) for filling a fluidic material, wherein the bag (1, 2) comprises a bag wall (4) formed by at least two wall parts (5) connected to each other by at least one wall connection (6), and at least one flexible bag tube (7) arranged in the bag wall (4), through which the fluidic material can be fed into the bag (1, 2) or can be fed out of the bag (1, 2), wherein the wall connection (6) comprises at least one plastic-sealed seam (8), wherein the bag wall (4) is formed from a multilayer film, wherein the bag wall (4) comprises an outer layer made of polyethylene terephthalate (PET) and an inner layer made of a thermoplastic, namely polyethylene (PE), wherein the flexible bag tube (7) is arranged in a fluid-tight manner between two wall parts (5) in the seam (8), wherein the bag tube (7) is formed from a tube material which is soft compared to the material of the wall parts, namely from a thermoplastic elastomer (TPE), wherein the bag tube (7) is arranged in the seam (8) by heat sealing, namely by a PE-on-TPE sealed seam, and wherein the material, namely a thermoplastic elastomer (TPE), from which the tube is formed, is so soft that the tube can be compressed with a pressing force acting on the outer circumferential surface of the tube in order to seal the tube, wherein the pressing force is less than 10 N, so that the pressing force can be applied by two fingers of a hand,
and wherein the bag is formed as a double bag (2) having two bag segments (3) or as a multi bag having a plurality of bag segments (3), wherein each bag segment (3) is assigned in each case one bag tube (7).

2. The gas analysis bag (1, 2) according to claim 1, wherein the bag tube (7) comprises a styrene-ethylene-butylene block copolymer (SEB).

3. The gas analysis bag (1, 2) according to claim 1 or 2, wherein the bag wall (4) comprises a barrier layer, in particular made of a metal, preferably aluminum.

4. The gas analysis bag (1, 2) according to any one of claims 1 to 3 having a closure which applies in particular a pressing force and which is preferably arranged in or on the bag tube (7).

5. A package for packaging fluidic material, having a package housing which comprises in particular paper and/or cardboard, and having at least one bag (1, 2) according to any one of claims 1 to 4 which is captively arranged in the package housing.

6. A method for making a bag according to claim 1 or according to claim 3 when dependent on claim 1, or according to claim 4 when dependent on either claim 1 or 3, in which a first wall part comprising a PE layer is connected to at least one second wall part comprising a PE layer to form a bag wall by heat sealing the PE layers of the wall parts along a sealed seam forming a wall connection, wherein the bag wall (4) comprises an outer layer made of polyethylene terephthalate (PET) and an inner layer made of PE, in which, through a tube connection arranged in the region of the wall connection, a flexible bag tube formed from a tube material which is soft compared with the material of the wall parts is connected to the PE layer of the first bag wall and/or to the PE layer of the second bag wall by heat sealing so that the bag, in particular in the region of the wall connection and in the region of the tube connection, is gas-tight or almost gas-tight, wherein the tube is formed from a thermoplastic elastomer (TPE), and wherein the wall connection is interrupted in the region of the tube connection in such a manner that the wall connection in the region of the tube connection is replaced by the tube connection.

7. The method for making a bag according to claim 2 or according to claim 3 when dependent on claim 2, or according to claim 4 when dependent on either claim 2 or 3, in which a first wall part comprising a PE layer is connected to at least one second wall part comprising a PE layer to form a bag wall by heat sealing the PE layers of the wall parts along a sealed seam forming a wall connection, wherein the bag wall (4) comprises an outer layer made of polyethylene terephthalate (PET) and an inner layer made of PE, in which, through a tube connection arranged in the region of the wall connection, a flexible bag tube formed from a tube material which is soft compared with the material of the wall parts is connected to the PE layer of the first bag wall and/or to the PE layer of the second bag wall by heat sealing so that the bag, in particular in the region of the wall connection and in the region of the tube connection, is gas-tight or almost gas-tight, wherein the tube is formed from a thermoplastic elastomer (TPE) and comprises a styrene-ethylene-butylene block copolymer (SEB), and wherein the wall connection is interrupted in the region of the tube connection in such a manner that the wall connection in the region of the tube connection is replaced by the tube connection.

8. A method for transporting a fluid sample, in particular a gas sample, in which the fluid sample is filled into a bag according to any one of claims 1 to 4 or into a package according to claim 5, and in which the bag is subsequently sealed so that the fluid sample can be transported in the sealed bag.

9. A method for analyzing a fluid sample, in which a fluid sample is produced or obtained, in which the fluid sample is filled into a bag according to any one of claims 1 to 4 or into a package according to claim 5, in which the filled bag is transported to an analyzing device according to a method according to claim 8, in which a bag tube arranged in the bag is fluidically connected to the analyzing device so that the fluid can be supplied to the analyzing device, and in which the fluid can be analyzed in the fluid device.

## Revendications

1. Sachet d'analyse de gaz (1,2) pour le remplissage d'un matériau pouvant s'écouler, sachant que le sachet (1, 2) comprend une paroi de sachet (4) formée par au moins deux parties de paroi (5) reliées entre elles par au moins une liaison de paroi (6), ainsi qu'au moins un tuyau de sachet (7) flexible disposé dans la paroi de sachet (4), à travers lequel le matériau pouvant s'écouler peut être introduit dans le sachet (1, 2) ou sorti du sachet (1, 2), sachant que la liaison de paroi (6) comprend au moins un joint scellé de matière plastique (8), sachant que la paroi de sachet (4) est formée d'une feuille multicouche, sachant que la paroi de sachet (4) comprend une couche extérieure de polyéthylène téréphtalate (PET) et une couche intérieure de matière thermoplastique, notamment de polyéthylène (PE), sachant que le tuyau de sachet flexible (7) est disposé étanche au fluide entre deux parties de paroi (5) dans le joint (8), sachant que le tuyau de sachet (7) est formé d'un matériau de tuyau mou comparé au matériau des parties de paroi, notamment d'un élastomère thermoplastique (ETP), sachant que le tuyau de sachet (7) est disposé dans le joint (8) par thermoscellage, notamment par un joint scellé PE sur ETP et sachant que le matériau, notamment un élastomère thermoplastique (ETP), à partir duquel le tuyau est formé, est mou de telle manière que le tuyau peut être compressible fermant le tuyau avec une force de compression agissant sur la surface d'enveloppe extérieure du tuyau, sachant que la force de compression est inférieure à 10 N de telle manière que la force de compression peut être appliquée par les deux doigts d'une main,
et sachant que le sachet est formé en tant que sachet double (2) avec deux segments de sachet (3) ou en tant que sachet multiple avec plusieurs segments de sachet (3), sachant qu'un tuyau de sachet (7) est respectivement attribué à chaque segment de sachet (3).

2. Sachet d'analyse de gaz (1, 2) selon la revendication 1, sachant que le tuyau de sachet (7) comprend un copolymère bloc styrol-éthylène-butylène (SEB).

3. Sachet d'analyse de gaz (1, 2) selon la revendication 1 ou 2, sachant que la paroi de sachet (4) comprend une couche d'arrêt, en particulier en métal de préférence en aluminium.

4. Sachet d'analyse de gaz (1, 2) selon l'une quelconque des revendications 1 à 3, avec en particulier une fermeture appliquant en particulier une force de compression, qui est disposée de préférence dans ou sur le tuyau de sachet (7).

5. Emballage pour emballer du matériau pouvant s'écouler avec une enveloppe d'emballage, laquelle comprend en particulier du papier et/ou du carton et avec au moins un sachet (1, 2) disposé de façon imperdable dans l'enveloppe d'emballage selon l'une quelconque des revendications 1 à 4.

6. Procédé de fabrication d'un sachet selon la revendication 1 ou selon la revendication 3, si dépendante de la revendication 1, ou selon la revendication 4, si dépendante d'une quelconque des revendications 1 ou 3, pour lequel une première partie de paroi comprenant une couche de PE avec au moins une deuxième partie de paroi comprenant une couche de PE est reliée à une paroi de sachet par thermoscellage des couches de PE des parties de paroi le long d'un joint scellé formant une liaison de paroi, sachant que la paroi de sachet (4) comprend une couche extérieure de polyéthylène téréphtalate (PET) et une couche intérieure de PE pour lequel un tuyau de sachet flexible dans un matériau de tuyau mou comparé au matériau des parties de paroi formée par une liaison de tuyau disposée dans la zone de la liaison de paroi, est relié à la couche de PE de la première paroi de sachet et/ou à la couche de PE de la deuxième paroi de sachet par thermoscellage, de telle sorte que le sachet, en particulier dans la zone de liaison de paroi et dans la zone de la liaison de tuyau, est étanche au gaz ou presque étanche au gaz, sachant que le tuyau est formé d'un élastomère thermoplastique (ETP) et sachant que la liaison de paroi dans la zone de la liaison de tuyau est interrompue de telle sorte que la liaison de paroi dans la zone de la liaison de tuyau est remplacée par la liaison de tuyau.

7. Procédé de fabrication d'un sachet selon la revendication 2 ou selon la revendication 3, si dépendante de la revendication 2, ou selon la revendication 4, si dépendante d'une quelconque des revendications 2 ou 3, pour lequel une première partie de paroi comprenant une couche de PE avec au moins une deuxième partie de paroi comprenant une couche de PE est reliée à une paroi de sachet par thermoscellage des couches de PE des parties de paroi le long d'un joint scellé formant une liaison de paroi, sachant que la paroi de sachet (4) comprend une couche extérieure de polyéthylène téréphtalate (PET) et une couche intérieure de PE pour lequel un tuyau de sachet flexible dans un matériau de tuyau mou comparé au matériau des parties de paroi formé par une liaison de tuyau disposée dans la zone de la liaison de paroi, est relié à la couche de PE de la première paroi de sachet et/ou à la couche de PE de la deuxième paroi de sachet par thermoscellage, de telle sorte que le sachet, en particulier dans la zone de liaison de paroi et dans la zone de la liaison de tuyau, est étanche au gaz ou presque étanche au gaz, sachant que le tuyau est formé d'un élastomère thermoplastique (ETP) et comprend un copolymère bloc styrol-éthylène-butylène (SEB),
et sachant que la liaison de paroi dans la zone de la liaison de tuyau est interrompue de telle manière que la liaison de paroi dans la zone de la liaison de tuyau est remplacée par la liaison de tuyau.

8. Procédé de transport d'un échantillon fluide, en particulier d'un échantillon de gaz, pour lequel l'échantillon de gaz est introduit dans un sachet selon l'une quelconque des revendications 1 à 4 ou dans un emballage selon la revendication 5 et pour lequel le sachet est ensuite fermé de telle manière que l'échantillon fluide peut être transporté dans le sachet fermé.

9. Procédé d'analyse d'un échantillon fluide, pour lequel un échantillon fluide est produit ou créé, pour lequel l'échantillon fluide est introduit dans un sachet selon l'une quelconque des revendications 1 à 4 ou dans un emballage selon la revendication 5, pour lequel le sachet rempli est transporté vers un dispositif d'analyse, selon un procédé selon la revendication 8, pour lequel un tuyau de sachet disposé dans le sachet est relié selon la technique des fluides au dispositif d'analyse de telle manière que le fluide peut être acheminé dans le dispositif d'analyse et pour lequel le fluide peut être analysé dans le dispositif d'analyse.
